# EUROPEAN PATENT SPECIFICATION

(11) **EP 1 151 117 B1**
(45) Date of publication and mention of the grant of the patent: **15.10.2008**
(21) Application number: 00905955.1
(22) Date of filing: 02.02.2000
(51) Int. Cl.: C12N 15/62, C12N 15/63, C12N 15/70, C12P 21/00, C12P 21/04, C07K 19/00, C07K 1/113, C07K 1/14, C07K 1/22

(54) **INTEIN-MEDIATED PROTEIN LIGATION OF EXPRESSED PROTEINS**
INTEIN-VERMITTELTE LIGATION VON EXPRIMIERTEN PROTEINEN
LIGATION DE PROTEINES EXPRIMEES INDUITE PAR INTEINES

(30) Priority: 12.02.1999 US 249543
(43) Date of publication of application: 07.11.2001
(73) Proprietor: NEW ENGLAND BIOLABS, INC., Beverly Massachusetts 01915 (US); Evans, Thomas C., Somerville, MA 02143 (US); Xu, Ming-Qun, Hamilton, MA 01982 (US)
(72) Inventor: EVANS, Thomas C., Somerville, MA 02143 (US); XU, Ming-Qun, Hamilton, MA 01982 (US)
(74) Representative: Froud, Clive
(86) International application number: PCT/US2000/002764
(87) International publication number: WO 2000/047751

(56) References cited:
- WO-A-00/18881
- US-A- 5 834 247
- EVANS T C ET AL: "Semisynthesis of cytotoxic proteins using a modified splicing element" PROTEIN SCIENCE, CAMBRIDGE UNIVERSITY PRESS, CAMBRIDGE, GB, vol. 7, 1998, pages 2256-2264, XP002925638 ISSN: 0961-8368
- SEVERINOV K ET AL: "Expressed protein ligation, a novel method for studying protein-protein interactions in transcription" JOURNAL OF BIOLOGICAL CHEMISTRY, AMERICAN SOCIETY OF BIOLOGICAL CHEMISTS, BALTIMORE, MD, US, vol. 273, no. 26, 26 June 1998 (1998-06-26), pages 16205-16209, XP002238975 ISSN: 0021-9258
- THOMAS C EVANS ET AL: "The in vitro Ligation of bacterially expressed proteins Using an Intein from Methanobacterium thermoautotrophicum" JOURNAL OF BIOLOGICAL CHEMISTRY, THE AMERICAN SOCIETY OF BIOLOGICAL CHEMISTS, INC.,, US, vol. 274, no. 7, 12 February 1999 (1999-02-12), pages 3923-3926, XP002986645 ISSN: 0021-9258
- MATHYS S ET AL: "Characterization of a self-splicing mini-intein and its conversion into autocatalytic N- and C-terminal cleavage elements: facile production of protein building blocks for protein ligation" GENE, ELSEVIER BIOMEDICAL PRESS. AMSTERDAM, NL, vol. 231, no. 1-2, 29 April 1999 (1999-04-29), pages 1-13, XP004166772 ISSN: 0378-1119
- XU. R. ET. AL.: 'Chemical ligation of folded recombinant proteins: Segmental isotopic labeling of domains for NMR studies' PROCEEDINGS OF THE NATIONAL ACADEMY OF SCIENCES, USA vol. 96, no. 2, January 1999, pages 388 - 393, XP002928538
- WU, H. ET. AL.: 'Protein trans-splicing and functional mini-inteins of a cyanobacterial dnB intein' BIOCHIMICA ET BIOPHYSICA ACTA. vol. 1387, no. 1-2, September 1998, pages 422 - 432, XP002928539
- WU, H. ET. AL.: 'Protein trans-splicing by a split intein encoded in a split DnaE gene of Synechocysitics sp. PCC6803' PROCEEDINGS OF THE NATIONAL ACADEMY OF SCIENCES, USA. vol. 95, no. 15, August 1998, pages 9226 - 9231, XP002928540
- SMITH, D. R. ET. AL.: 'Complete Genome Sequence of Methanobacterium thermoautotrophicum (delta)H: Functional Analysis and Comparative Genomics' JOURNAL OF BACTERIOLOGY vol. 179, no. 22, November 1997, pages 7135 - 7155, XP002928541
- CHONG S. ET. AL.: 'Utilizing the C-terminal cleavage activity of a protein splicing element to purify recombinant proteins in a single chromatographic step' NUCLEIC ACIDS RESEARCH vol. 26, no. 22, 15 November 1998, pages 5109 - 5115, XP002928542

## Description

### BACKGROUND OF THE INVENTION

The present invention relates to methods of intein-mediated ligation of proteins. More specifically, the present invention relates to intein-mediated ligation of expressed proteins containing a predetermined N-terminal residue and/or a C-terminal thioester generated via use of one or more naturally occurring or modified inteins. Preferably, the predetermined residue is cysteine.

Inteins are the protein equivalent of the self-splicing RNA introns (see Perler et al., Nucleic Acids Res. 22:1125-1127 (1994)), which catalyze their own excision from a precursor protein with the concomitant fusion of the flanking protein sequences, known as exteins (reviewed in Perler et al., Curr. Opin. Chem. Biol. 1:292-299 (1997); Perler, F. B. Cell 92(1):1-4 (1998); Xu et al.,EMBO J. 15(19):5146-5153 (1996)).

Studies into the mechanism of intein splicing led to the development of a protein purification system that utilized thiol-induced cleavage of the peptide bond at the N-terminus of the *Sce* VMA intein (Chong et al., Gene 192(2):271-281 (1997)). Purification with this intein-mediated system generates a bacterially-expressed protein with a C-terminal thioester (Chong et al., (1997)). In one application, where it is described to isolate a cytotoxic protein, the bacterially expressed protein with the C-terminal thioester is then fused to a chemically-synthesized peptide with an N-terminal cysteine using the chemistry described for "native chemical ligation" (Evans et al., Protein Sci. 7:2256-2264 (1998); Muir et al., Proc. Natl. Acad. Sci. USA 95:6705-6710 (1998)).

This technique, referred to as "intein-mediated protein ligation" (IPL), represents an important advance in protein semisynthetic techniques. However, because chemically-synthesized peptides of larger than about 100 residues are difficult to obtain, the general application of IPL is limited by the requirement of a chemically-synthesized peptide as a ligation partner.

IPL technology would be significantly expanded if an expressed protein with a predetermined N-terminus, such as cysteine, could be generated. This would allow the fusion of one or more expressed proteins from a host cell, such as bacterial, yeast or mammalian cells.

One method of generating an N-terminal cysteine is with the use of proteases. However, proteases have many disadvantages, such as the possibility of multiple protease sites within a protein, as well as the chance of non-specific degradation. Furthermore, following proteolysis, the proteases must be inactivated or purified away from the protein of interest before proceeding with IPL. (Xu, et al., Proc. Natl. Acad. Sci. USA 96(2):388-393 (1999) and Erlandson, et al., Chem. Biol., 3 :981-991 (1996)).

There is, therefore, a need for an improved intein-mediated protein ligation method which overcomes the noted limitations of current IPL methods and which eliminates the need for use of proteases to generate an N-terminal cysteine residue. Such an improved IPL method would have widespread applicability for the ligation of expressed proteins, for example, labeling of extensive portions of a protein for, among other things, NMR analysis.

### SUMMARY OF THE INVENTION

In one embodiment, the present invention provides a method for ligating target proteins, **characterized in that** it comprises:
(a) expressing from a first plasmid a first fusion protein comprising a first target protein having a C-terminus fused to an intein;
(b) expressing from a second plasmid a second fusion protein comprising a second target protein having an N-terminal cysteine fused to an intein;
(c) obtaining a preparation of the first fusion protein and a preparation of the second fusion protein;
(d) adding a thiol reagent to the preparation of the first fusion protein so that the first intein is cleaved so as to form a C-terminal thioester on the first target protein;
(e) cleaving the second intein from the second target protein in the preparation of the second fusion protein and forming an N-terminal cysteine on the second target protein;
   and
(f) permitting ligation of the first target protein having the C-terminal thioester with the second target protein of (e);
   the inteins displaying either N- and/or C-terminal cleavage.

In another embodiment, the present invention provides a method for cyclization of a target protein having an N-terminal cysteine, **characterized in that** it comprises:
(a) expressing from a plasmid in a host cell, a fusion protein comprising a target protein having a cysteine at the N-terminus, and two inteins, the first intein being fused to the C-terminus of the target protein and the second intein being fused to the N-terminal cysteine of the target protein;
(b) obtaining a preparation of the expressed fusion protein;
(c) inducing cleavage of the fusion protein, comprising addition of a thiol reagent, to remove the first and second inteins from the target protein thereby obtaining the target protein having a C-terminal thioester and an N-terminal cysteine;
   and
(d) permitting intramolecular ligation of the N-terminus of the target protein to the C-terminus of the target protein thereby forming a cyclised protein;
   the inteins displaying either N- and/or C-terminal cleavage.

In a further embodiment, the present invention provides a method for forming a polymer by intermolecular ligation between target proteins in a preparation, characterized in that it comprises:
(a) forming each target protein having a C-terminal thioester and an N-terminal cysteine by cleaving a first and second intein from a fusion protein, the fusion protein comprising a target protein fused to the first intein at the C-terminal end and the second intein at the N-terminal end;
   and
(b) allowing intermolecular ligation between target proteins by reacting the C-terminal thioester of one target protein with the N-terminal cysteine at the N-terminus of another target protein to form a polymer;
   the inteins displaying either N- and/or C-terminal cleavage.

In yet another embodiment, the present invention provides a method for generating a cysteine at the N-terminus of a target protein, **characterized in that** it comprises:
expressing in a host cell, a nucleic acid encoding a fusion protein comprising an intein and a target protein wherein the intein-encoding sequence is 5' to a codon specifying a cysteine at the amino terminus of the target protein;
   and
cleaving the intein from the target protein so as to generate the cysteine at the N-terminus of the target protein;
   the inteins displaying either N- and/or C-terminal cleavage.

In a still further embodiment, the present invention provides a method for ligating a first and a second target protein, **characterized in that** it comprises:
(a) inducing cleavage of a first intein from a fusion protein comprising the intein and a first target protein, to form an N-terminus cysteine on the target protein;
(b) combining in a mixture the first target protein of (a) with a second target protein having a C-terminus thioester;
   and
(c) ligating the first and second target proteins;
   the inteins displaying either N- and/or C-terminal cleavage.

In another embodiment, the present invention provides a method for cyclization of a target protein having an N-terminal cysteine, **characterized in that** it comprises:
adding a thiol reagent to a fusion protein comprising a target protein having an N-terminal cysteine and an intein fused to the C-terminus of the target protein in order to induce cleavage of the intein from the target protein and the formation of a C-terminal thioester on the target protein;
   and
permitting intramolecular ligation of the N-terminus of the C-terminal thioester of the target protein to the N-terminal cysteine of the target protein for cyclization of the target protein;
   the inteins displaying either N- and/or C-terminal cleavage.

In a further embodiment, the present invention provides a further modified Mth RIR1 intein, **characterized in that** the intein comprising the amino acid sequence depicted in SEQ ID NO: 24 comprises a substitution of alanine for the asparagine at position 134 at the C-terminus of the intein or a substitution of alanine or serine for the cysteine at position 1 at the N-terminus of the intein.

Having indicated the scope of the present invention, it will now be further described and illustrated in context in more general terms.

In accordance with the present invention, there is provided a method for the ligation of expressed proteins utilizing one or more inteins which display cleavage at their N- and/or C-termini. In accordance with the present invention, such inteins may occur either naturally or may be modified to cleave at their N- and/or C-termini. Inteins displaying N- and/or C-terminal cleavage enable the facile isolation of a protein having a C-terminal thioester and a protein having an N-terminal amino acid residue such as cysteine, respectively, for use in the fusion of one or more expressed proteins. Alternatively, the method may be used to generate a single protein having both a C-terminal thioester and a specified N-terminal amino acid residue, such as cysteine, for the creation of cyclic or polymerized proteins. These methods involve the steps of generating at least one C-terminal thioester-tagged first target protein, generating at least one second target protein having a specified N-terminal amino acid residue, for example cysteine, and ligating these proteins. This method may be used where a single protein is expressed, where, for example, the C-terminal thioester end of the protein is fused to the N-terminal end of the same protein. The method may further include chitin-resin purification steps.

In one preferred embodiment the intein from the RIR1 *Methanobacterium thermoautotrophicum* is modified to cleave at either the C-terminus or N-terminus. The modified intein allows for the release of a bacterially expressed protein during a one-column purification, thus eliminating the need proteases entirely. DNA encoding these modified inteins and plasmids containing these modified inteins are also provided by the instant invention.

### BRIEF DESCRIPTION OF THE DRAWINGS

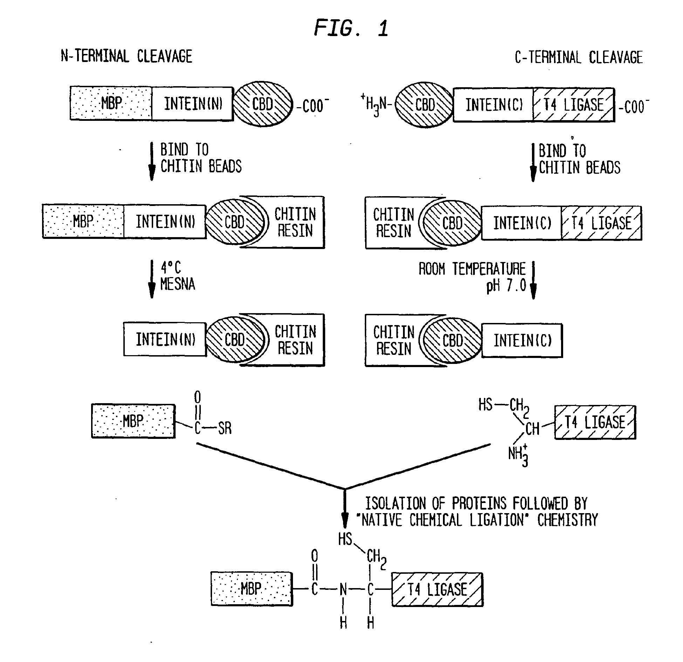

Figure 1 is a diagram depicting both the N-terminal and C-terminal cleavage reactions which comprise intein-mediated protein ligation. The modified *Mth* RIR1 intein was used to purify both MBP with a C-terminal thioester and T4 DNA ligase with an N-terminal cysteine. The *Mth* RIR1 intein for N-terminal cleavage, intein(N), carried the P⁻¹G/N¹³⁴A double mutation. The full length fusion protein consisting of MBP-intein(N)-CBD was separated from cell extract by binding the CBD portion of the fusion protein to a chitin resin. Overnight incubation in the presence of 100 mM 2-mercaptoethanesulfonic acid (MESNA) induced cleavage of the peptide bond prior to the N-terminus of the intein and created a thioester on the C-terminus of MBP. The C-terminal cleavage vector, intein(C), had the P⁻¹G/C¹A double mutation. The precursor CBD-intein(C)-T4 DNA ligase was isolated from induced *E. coli* cell extract by binding to a chitin resin as described for N-terminal cleavage. Fission of the peptide bond following the C-terminal residue of the intein at a preferred temperature and pH resulted in the production of T4 DNA ligase with an N-terminal cysteine. Ligation occurred when the proteins containing the complementary reactive groups were mixed and concentrated, resulting in a native peptide bond between the two reacting species.

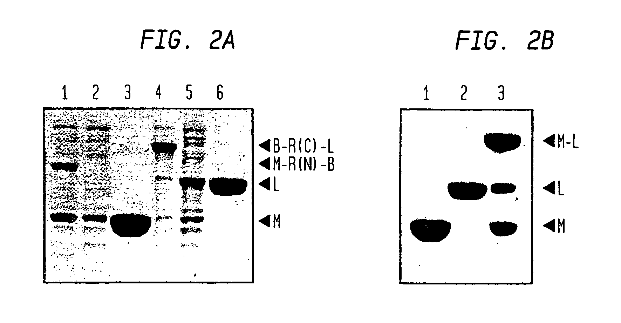

Figure 2A is a gel depicting the purification of a C-terminal thioester-tagged maltose binding protein (MBP) via a thiol-inducible *Mth* RIR1 intein construct pMRB10G (containing the modified intein, R(N), with P⁻¹G/N¹³⁴A mutation) and the purification of T4 DNA ligase having an N-terminal cysteine using the vector pBRL-A (containing the modified intein, R(C), with P-¹G/C¹A mutation). Lanes 1-3, purification of maltose binding protein (MBP) (M, 43 kDa) with a C-terminal thioester. Lane 1. ER2566 cells transformed with plasmid pMRB10G following Isopropyl β-D-thiogalactopyranoside (IPTG) induction. Lane 2. Cell extract after passage over a chitin resin. Note that the fusion protein, M-R(N)-B, binds to the resin, where B is the chitin binding domain. Lane 3. Fraction 3 of the elution from the chitin resin following overnight incubation at 4°C in the presence of 100 mM MESNA. Lanes 4-6, purification of T4 DNA ligase (L, 56 kDa) with an N-terminal cysteine. Lane 4. IPTG induced ER2566 cells containing plasmid pBRL-A. Lane 5. Cell extract after application to a chitin resin. B-R(C)-L, the fusion protein, binds to the resin. Lane 6. Elution of T4 DNA ligase with an N-terminal cysteine after overnight incubation at room temperature in pH 7 buffer
Figure 2B is a gel depicting ligation of T4 DNA ligase having an N-terminal cysteine to a C-terminal thioester tagged MBP. Lane 1. Thioester-tagged MBP. Lane 2. T4 DNA ligase with an N-terminal cysteine. Lane 3. Ligation reaction of MBP (0.8 mM) with T4 DNA ligase (0.8 mM), generating M-L, after overnight incubation at 4°C.

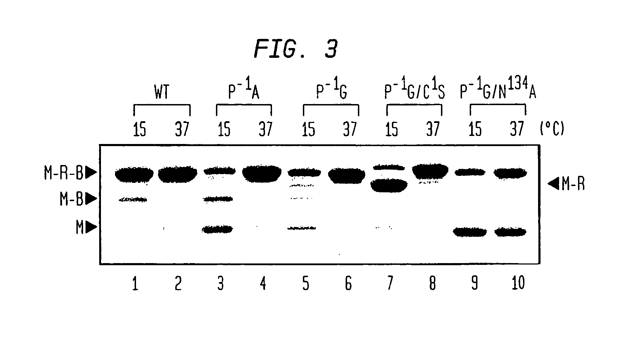

Figure 3 is a gel depicting the effect of induction temperature on the cleaving and/or splicing activity of the *Mth* RIR1 intein or *Mth* RIR1 intein mutants. The *Mth* RIR1 intein or mutants thereof, with 5 native N- and C-terminal extein residues were induced at either 15°C or 37°C. The intein was expressed as a fusion protein (M-R-B, 63 kDa) consisting of N-terminal maltose binding protein (M, 43 kDa), the *Mth* RIR1 intein (R, 15 kDa) and at its C-terminus was the chitin binding domain (B, 5 kDa). Lanes 1 and 2. M-R-B with the unmodified *Mth* RIR1 intein. Note the small amount of spliced product (M-B, 48 kDa). Lanes 3 and 4. *Mth* intein with Pro⁻¹ replaced with Ala, M-R-B(P⁻¹A). Both spliced product (M-B) and N-terminal cleavage product (M) are visible. Lanes 5 and 6. Replacement of Pro⁻¹ with Gly (M-R-B(P⁻¹G)) showed some splicing as well as N- and C-terminal cleavage, M and M-R, respectively. Lanes 7 and 8. The Pro⁻¹ to Gly and Cys¹ to Ser double mutant, M-R-B(P⁻¹G/C¹S), displayed induction temperature dependent C-terminal cleavage (M-R) activity. Lanes 9 and 10. The M-R-B(P⁻¹G/N¹³⁴A) mutant possessed only N-terminal cleavage activity producing M. The *Mth* intein or *Mth* intein -CBD fusion is not visible in this Figure.

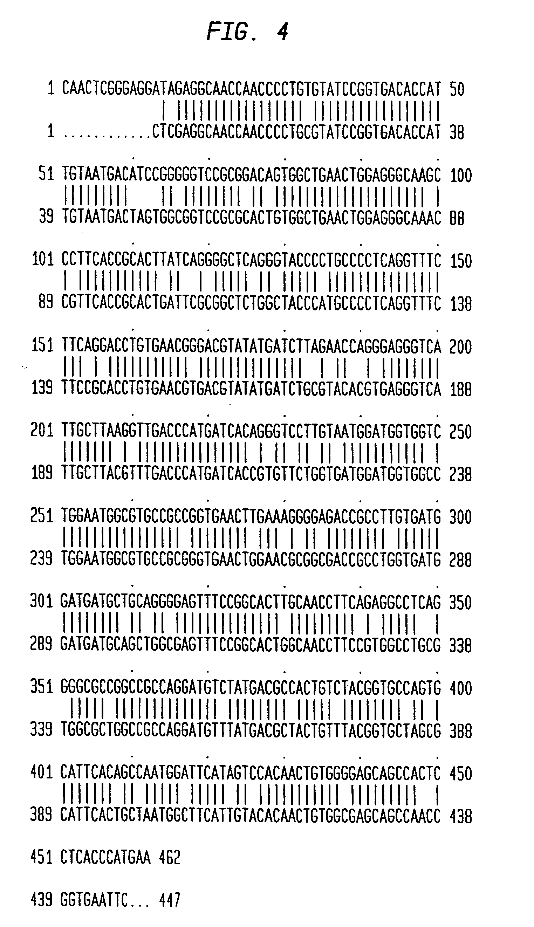

Figure 4 is a nucleotide sequence (SEQ ID NO:23) comparison of wild type *Mth* RIR1 intein and synthetic *Mth* RIR1 intein indicating the location of 61 silent base mutations designed to increase expression in *E. coli.* DNA alignment of the wild type *Mth* RIR1 intein (top strand) and the synthetic *Mth* RIR1 intein (bottom strand). To increase expression levels in *E. coli,* 61 silent base changes were made in 49 seperate codons when creating the synthetic gene. The first and last codons of the wild type *Mth* RIR1 intein are shown in bold.

### DETAILED DESCRIPTION

The present invention provides a solution to the limitations of current intein-mediated ligation methods by eliminating the need for a synthetic peptide as a ligation partner, and providing a method which is suitable for the fusion one or more expressed proteins.

In general, any intein displaying N- and/or C-terminal cleavage at its splice junctions can be used to generate a defined N-terminus, such as cysteine as well as a C-terminal thioester for use in the fusion of expressed proteins. Inteins which may be used in practicing the present invention include those described in Perler, et al., Nucleic Acids Res., 27(1):346-347 (1999).

In accordance with one preferred embodiment, an intein found in the ribonucleoside diphosphate reductase gene of *Methanobacterium thermoautotrophicum* (the *Mth* RIR1 intein) was modified for the facile isolation of a protein with an N-terminal cysteine for use in the *in vitro* fusion of two bacterially-expressed proteins. The 134-amino acid *Mth* RIR1 intein is the smallest of the known mini-inteins, and may be close to the minimum amino acid sequence needed to promote splicing (Smith et. al., J. Bacteriol. 179: 7135-7155 (1997)).

The *Mth* RIR1 intein has a proline residue on the N-terminal side of the first amino acid of the intein. This residue was previously shown to inhibit splicing in the Sce VMA intein (Chong et al., J. Biol. Chem. 273:10567-10577 (1998)). The intein was found to splice poorly in *E. coli* when this naturally occurring proline is present. Splicing proficiency increases when this proline is replaced with an alanine residue. Constructs that display efficient N- and C-terminal cleavage are created by replacing either the C-terminal asparagine or N-terminal cysteine of the intein, respectively, with alanine.

These constructs allow for the formation of an intein-generated C-terminal thioester on a first target protein and an intein-generated N-terminal cysteine on a second target protein. These complementary reactive groups may then be ligated via native chemical ligation to produce a peptide bond (Evans et al *supra* (1998), Muir et al *supra* (1998)). Alternatively, a single protein containing both reactive groups may be generated for the creation of cyclic or polymerized proteins. Likewise, more than one first or second target proteins may be generated via use of multiple mutant inteins.

As used herein, the terms fusion and ligation are used interchangeably. Also as used herein, protein shall mean any protein, fragment of any protein, or peptide capable of ligation according to the methods of the instant invention. Further, as used herein, target protein shall mean any protein the ligation of which, according to the methods of the instant invention, is desired.

The general method of intein-mediated protein ligation in accordance with the present invention is as follows:
(1) An intein of interest is isolated and cloned into an appropriate expression vector(s) such as bacterial, plant, insect, yeast and mammalian cells.
(2) The intein is engineered for N- and/or C-terminal cleavage unless the wild type intein displays the desired cleavage activities. In a preferred embodiment, a modified intein with the desired cleavage properties can be generated by substituting one or more residues within and/or flanking the intein sequence. For example, a modified intein having N-terminal cleavage activity can be created by changing the last intein residue. Alternatively, a modified intein with C-terminal cleavage activity can be created by changing the first intein residue.
(3) The intein with N- and/or C-terminal cleavage activity is fused with an affinity tag to allow purification away from other endogenous proteins.
(4) The intein or inteins, either wild type or modified, that display N-terminal and/or C-terminal cleavage, or both, are fused to the desired target protein coding region or regions upstream and/or downstream of the intein.
(5) An intein that cleaves at its N-terminus in a thiol reagent dependent manner is used to isolate a protein with a C-terminal thioester. This cleavage and isolation is, for example, carried out as previously described for the *Sce* VMA and *Mxe* GyrA inteins (Chong et al., Gene 192(2):271-281 (1997); Evans et al., Protein Sci. 7:2256-2264 (1998)). As discussed previously, multiple C-terminal thioester-tagged proteins may be generated at this step.
(6) A target protein having a specified N-terminus is generated by cleavage of a construct containing an intein that cleaves at its C-terminus. The specified N-terminal residue may be any of the amino acids, but preferably cysteine. As discussed previously, this step may alternately generate a specified N-terminal on the same protein containing a C-terminal thioester, to yield a single protein containing both reactive groups. Alternatively, multiple proteins having the specified N-terminus may be generated at this step.
(7) Thioester-tagged target protein and target protein having a specified N-termini are fused via intein-mediated protein ligation (IPL) (see Figure 2B). In a preferred embodiment, the N-terminus is cysteine. Alternatively, a single protein containing both a C-terminal thioester and a specified N-terminus, such as a cysteine, may undergo intramolecular ligation to yield a cyclic product and/or intermolecular ligation to yield polymerized proteins.

The methodology described by the instant invention significantly expands the utility of current IPL methods to enable the labeling of extensive portions of a protein for NMR analysis and the isolation of a greater variety of cytotoxic proteins. In addition, this advance opens the possibility of labeling the central portion of a protein by ligating three or more fragments.

The use of an intein or inteins with N-terminal and C-terminal cleavage activity provides the potential to create a defined N-terminus, such as a cysteine, and a C-terminal thioester on a single protein. The intramolecular ligation of the resulting protein generates a circular protein, whereas the intermolecular ligation of several of these proteins generates a protein polymer.

Cleavage at the N- and/or the C-terminus of an intein can be brought about by introducing changes to the intein and/or its extein sequences. Also, naturally occuring inteins may display these properties and require no manipulation. Cleavage at the N-and/or C-terminus of an intein can occur uncontrollably or induced using nucleophilc compounds, such as thiol reagents, temperature, pH, salt, chaotropic agents, or any combination of the aforementioned conditions and/or reagents.

The Examples presented below are only intended as specific preferred embodiments of the present invention and are not intended to limit the scope of the invention except as provided in the claims herein.

### EXAMPLE I

### Creation of the Mth RIR1 synthetic gene

The gene encoding the *Mth* RIR1 intein along with 5 native N- and C-extein residues (Smith et al. *supra* (1997)) was constructed using 10 oligonucleotides (New England Biolabs, Beverly, MA) comprising both strands of the gene, as follows:
1)
2)
3)
4)
5)
6)
7)
8)
9)
10)

To ensure maximal *E. coli* expression, the coding region of the synthetic *Mth* RIR1 intein incorporates 61 silent base mutations in 49 of the 134 codons (see Figure 4) in the wildtype *Mth* RIR1 intein gene (GenBank AE000845). The oligonucleotides were annealed by mixing at equimolar ratios (400 nM) in a ligation buffer (50 mM Tris-HCl, pH 7.5 containing 10 mM MgCl₂, 10 mM dithiothreitol, 1 mM ATP, and 25 *µ*g BSA) followed by heating to 95°C. After cooling to room temperature, the annealed and ligated oligonucleotides were inserted into the *Xho*I and *Age*I sites of pMYB5 (NEB), replacing the *Sce* VMA intein and creating the plasmid pMRB8P.

### Engineering the Mth RIR1 intein for N- and C-terminal cleavage

The unique *Xho*I and *Spe*I sites flanking the N-terminal splice junction and the unique *BsrG*I and *Age*I sites flanking the C-terminal splice junction allowed substitution of amino acid residues by linker replacement. The proline residue, Pro⁻¹, preceding the intein in pMRB8P was substituted with alanine or glycine to yield pMRB8A and pMRB8G1, respectively.

Substitution of Pro⁻¹Cys¹ with Gly-Ser or Gly-Ala yielded pMRB9GS and pMRB9GA, respectively. Replacing Asn¹³⁴ with Ala in pMRB8G1 resulted in pMRB10G. The following linkers were used for substitution of the native amino acids at the splice junctions (each linker was formed by annealing two synthetic oligonucleotides as described above):

| | |
|---|---|
| P⁻¹A linker: | |
| and | |
| P⁻¹G linker: | |
| and | |
| P⁻¹G/C¹S linker: | |
| and | |
| P⁻¹G/C¹A linker: | |
| and | |
| N¹³⁴A linker: | |
| and | |

pBRL-A was constructed by substituting the *Escherichia coli* maltose binding protein (MBP) and the *Bacillus circulans* chitin binding domain (CBD) coding regions in pMRB9GA with the CBD and the T4 DNA ligase coding regions, respectively, subcloned from the pBYT4 plasmid.

### EXAMPLE II

### Generating a thioester-tagged protein:

The pMRB10G construct from Example I contains the *Mth* RIR1 intein engineered to undergo thiol reagent induced cleavage at the N-terminal splice junction (Figure 1, N-terminal cleavage) and was used to isolate proteins with a C-terminal thioester as described previously for the *Sce* VMA and *Mxe* GyrA inteins (Chong et al. *supra* (1997); Evans et al., supra (1998)). Briefly, ER2566 cells (Evans et.al. (1998)) containing the appropriate plasmid were grown at 37°C in LB broth containing 100 *µ*g/mL ampicillin to an OD₆₀₀ of 0.5-0.6 followed by induction with IPTG (0.5 mM). Induction was either overnight at 15°C or for 3 hours at 30°C.

The cells were pelleted by centrifugation at 3,000xg for 30 minutes followed by resuspension in buffer A (20 mM Tris-HCl, pH 7.5 containing 500 mM NaCl). The cell contents were released by sonication. Cell debris was removed by centrifugation at 23,000xg for 30 minutes and the supernatant was applied to a column packed with chitin resin (10 mL bed volume) equilibrated in buffer A. Unbound protein was washed from the column with 10 column volumes of buffer A.

Thiol reagent-induced cleavage was initiated by rapidly equilibrating the chitin resin in buffer B (20 mM Tris-HCl, pH 8 containing 500 mM NaCl and 100 mM 2-mercaptoethane-sulfonic acid (MESNA)). The cleavage reaction, which simultaneously generates a C-terminal thioester on the target protein, proceeded overnight at 4°C after which the protein was eluted from the column. The use of the pMRB10G construct resulted in the isolation of MBP with a C-terminal thioester (Figure2A).

### Isolating proteins with an N-terminal cysteine

The pBRL-A construct from Example I contains an *Mth* RIR1 intein engineered to undergo controllable cleavage at its C-terminus, and was used to purify proteins with an N-terminal cysteine (Figure 1, C-terminal cleavage). The expression and purification protocol was performed as described in Example II, except with buffer A replaced by buffer C (20 mM Tris-HCl, pH 8.5 containing 500 mM NaCl) and buffer B replaced by buffer D (20 mM Tris-HCl, pH 7.0 containing 500 mM NaCl). Also, following equilibration of the column in buffer D the cleavage reaction proceeded overnight at room temperature.

The expression of plasmid pBRL-A resulted in the purification of 4-6 mg/L cell culture of T4 DNA ligase possessing an N-terminal cysteine (Figure 2A). Protein concentrations were determined using the Bio-Rad protein assay (Bio-Rad Laboratories, Inc., Hercules, CA).

### EXAMPLE III

### Protein-protein ligation using Intein-mediated Protein Ligation

Intein-mediated protein ligation (IPL) was used to fuse two proteins (Figure 2B). Freshly isolated thioester-tagged protein from Example II was mixed with freshly isolated protein containing an N-terminal cysteine residue from Example II, with typical starting concentrations of 1-200 *µ*M. The solution was concentrated with a Centriprep 3 or Centriprep 30 apparatus (Millipore Corporation, Bedford, MA) then with a Centricon 3 or Centricon 10 apparatus to a final concentration of 0.15-1.2 mM for each protein.

Ligation reactions proceeded overnight at 4°C and were visualized using SDS-PAGE with 12% Tris-glycine gels (Novex Experimental Technology, San Diego, CA) stained with Coomassie Brilliant Blue. Typical ligation efficiencies ranged from 20-60%.

### Confirmation of ligation in IPL reactions

A Factor Xa site in MBP that exists 5 amino acids N-terminal from the site of fusion (Maina et al, *supra* (1988)) allowed amino acid sequencing through the ligation junction. The sequence obtained was NH₂-TLEGCGEQPTGXLK-COOH (SEQ ID NO:21) which matched the last 4 residues of MBP (TLEG) followed by a linker sequence (CGEQPTG (SEQ ID NO:22)) and the start of T4 DNA ligase (ILK). During amino acid sequencing, the cycle expected to yield an isoleucine did not have a strong enough signal to assign it to a specific residue, so it was represented as an X. The cysteine was identified as the acrylamide alkylation product.

The Factor Xa proteolysis was performed on 2 mg of ligation reaction involving MBP and T4 DNA ligase. This reaction mixture was bound to 3 mL of amylose resin (New England Biolabs, Inc., Beverly, MA) equilibrated in buffer A (see Example II). Unreacted T4 DNA ligase was rinsed from the column with 10 column volumes of buffer A. Unligated MBP and the MBP-T4 DNA ligase fusion protein were eluted from the amylose resin using buffer E (20 mM Tris-HCl, pH 7.5 containing 500 mM NaCl and 10 mM maltose). Overnight incubation of the eluted protein with a 200:1 protein:bovine Factor Xa (NEB) ratio (w/w) at 4°C resulted in the proteolysis of the fusion protein and regeneration of a band on SDS-PAGE gels that ran at a molecular weight similar to T4 DNA ligase. N-terminal amino acid sequencing of the proteolyzed fusion protein was performed on a Procise 494 protein sequencer (PE Applied Biosystems, Foster City, CA).

### Temperature sensitivity of the Mth RIR1 intein

The cleavage and/or splicing activity of the *Mth* RIR1 intein was more proficient when protein synthesis was induced at 15°C than when the induction temperature was raised to 37°C (Figure 3). The effect temperature has on the *Mth* RIR1 represents a way to control the activity of this intein for use in controlled splicing or cleavage reactions. Replacement of Pro⁻¹ with a Gly and Cys¹ with a Ser resulted in a double mutant, the pMRB9GS construct, which showed only *in vivo* C-terminal cleavage activity when protein synthesis was induced at 15°C but not at 37°C. Another double mutant, the pMRB9GA construct, displayed slow cleavage, even at 15°C, which allowed the accumulation of substantial amounts of the precursor protein and showed potential for use as a C-terminal cleavage construct for protein purification.

## Claims

1. A method for ligating target proteins, **characterized in that** it comprises:
(a) expressing from a first plasmid a first fusion protein comprising a first target protein having a C-terminus fused to an intein;
(b) expressing from a second plasmid a second fusion protein comprising a second target protein having an N-terminal cysteine fused to an intein;
(c) obtaining a preparation of the first fusion protein and a preparation of the second fusion protein;
(d) adding a thiol reagent to the preparation of the first fusion protein so that the first intein is cleaved so as to form a C-terminal thioester on the first target protein;
(e) cleaving the second intein from the second target protein in the preparation of the second fusion protein and forming an N-terminal cysteine on the second target protein;
and
(f) permitting ligation of the first target protein having the C-terminal thioester with the second target protein of (e);
the inteins displaying either N- and/or C-terminal cleavage.

2. A method as claimed in claim 1 wherein the first intein is the Methanobacterium thermoautotrophicum (Mth) RIR1 intein depicted in SEQ ID NO: 24.

3. A method as claimed in claim 1 herein the second intein is the Mth RIR1 intein depicted in SEQ ID NO: 24.

4. A method as claimed in claim 2 or claim 3 wherein the further modification of the Mth RIR1 intein comprises a substitution of alanine for the asparagine at position 134 at the C-terminus or a substitution of alanine or serine for the cysteine at position 1 at the N-terminus.

5. A method as claimed in claim 1 wherein the second target protein of (e) is cleaved from the second intein in the presence of a thiol reagent or by modulating any of temperature, pH, salt, chaotropic agents or combinations thereof.

6. A method as claimed in claim 1 wherein (c) further comprises purifying the first or second fusion protein from the preparation.

7. A method as claimed in claim 6 wherein the purifying of the fusion protein further comprises binding to a chitin resin column.

8. A method as claimed in claim 1 wherein the first and second plasmids are capable of expression in a host cell selected from a bacterial, a yeast, a plant, an insect and a mammalian host cell.

9. A method for cyclization of a target protein having an N-terminal cysteine, **characterized in that** it comprises:
(a) expressing from a plasmid in a host cell, a fusion protein comprising a target protein having a cysteine at the N-terminus, and two inteins, the first intein being fused to the C-terminus of the target protein and the second intein being fused to the N-terminal cysteine of the target protein;
(b) obtaining a preparation of the expressed fusion protein;
(c) inducing cleavage of the fusion protein, comprising addition of a thiol reagent, to remove the first and second inteins from the target protein thereby obtaining the target protein having a C-terminal thioester and an N-terminal cysteine;
and
(d) permitting intramolecular ligation of the N-terminus of the target protein to the C-terminus of the target protein thereby forming a cyclized protein;
the inteins displaying either N- and/or C-terminal cleavage.

10. A method as claimed in claim 9 wherein the intein is the Mth RIR1 intein depicted in SEQ ID NO:24, or a further modified form of the Mth RIR1 intein wherein the modification comprises a substitution of alanine for the asparagine at position 134 at the C-terminus or a substitution of alanine or serine for the cysteine at position 1 at the N-terminus.

11. A method as claimed in claim 9 wherein (i) modulation of any of temperature, pH, salt, the concentration of chaotropic agents or combinations thereof cleaves the intein from the target protein to form the N-terminal cysteine and (ii) the addition of the thiol reagent forms the C-terminal thioester on the target protein.

12. A method claimed in claim 9 wherein (b) further comprises: purifying the fusion protein from the preparation.

13. A method as claimed in claim 9 wherein the purifying of the fusion protein further comprises binding to a chitin resin column.

14. A method as claimed in claim 9 wherein the plasmid is capable of expression in a host cell selected from a bacterial, a yeast, a plant, an insect and a mammalian host cell.

15. A method for forming a polymer by intermolecular ligation between target proteins in a preparation, **characterized in that** it comprises:
(a) forming each target protein having a C-terminal thioester and an N-terminal cysteine by cleaving a first and second intein from a fusion protein, the fusion protein comprising a target protein fused to the first intein at the C-terminal end and the second intein at the N-terminal end;
and
(b) allowing intermolecular ligation between target proteins by reacting the C-terminal thioester of one target protein with the N-terminal cysteine at the N-terminus of another target protein to form a polymer;
the inteins displaying either N- and/or C-terminal cleavage.

16. A method as claimed in claim 15 wherein the first intein is the Mth RIR1 intein depicted in SEQ ID NO:24.

17. A method as claimed in claim 15 wherein the second intein is the Mth RIR1 intein depicted in SEQ ID NO:24.

18. A method as claimed in claim 16 or claim 17 wherein the further modification of the Mth RIR1 intein comprises a substitution of alanine for the asparagine at position 134 at the C-terminus or a substitution of alanine or serine for the cysteine at position 1 at the N-terminus.

19. A method as claimed in claim 15 wherein the second intein is cleaved from the target protein by modulating temperature, pH, salt or chaotropic agents or combinations thereof.

20. A method as claimed in claim 15 wherein the fusion protein is expressed by a plasmid which is capable of expression in a host cell selected from a bacterial, a yeast, a plant, an insect and a mammalian host cell.

21. A method for generating a cysteine at the N-terminus of a target protein, **characterized in that** it comprises:
expressing in a host cell, a nucleic acid encoding a fusion protein comprising an intein and a target protein wherein the intein-encoding sequence is 5' to a codon specifying a cysteine at the amino terminus of the target protein;
and
cleaving the intein from the target protein so as to generate the cysteine at the N-terminus of the target protein;
the inteins displaying either N- and/or C-terminal cleavage.

22. A method for ligating a first and a second target protein, **characterized in that** it comprises:
(a) inducing cleavage of a first intein from a fusion protein comprising the intein and a first target protein, to form an N-terminus cysteine on the target protein;
(b) combining in a mixture the first target protein of (a) with a second target protein having a C-terminus thioester;
and
(c) ligating the first and second target proteins;
the inteins displaying either N- and/or C-terminal cleavage.

23. A method for cyclization of a target protein having an N-terminal cysteine, **characterized in that** it comprises:
adding a thiol reagent to a fusion protein comprising a target protein having an N-terminal cysteine and an intein fused to the C-terminus of the target protein in order to induce cleavage of the intein from the target protein and the formation of a C-terminal thioester on the target protein;
and
permitting intramolecular ligation of the N-terminus of the C-terminal thioester of the target protein to the N-terminal cysteine of the target protein for cyclization of the target protein;
the inteins displaying either N- and/or C-terminal cleavage.

24. A further modified Mth RIR1 intein, **characterized in that** the intein comprising the amino acid sequence depicted in SEQ ID NO:24 comprises a substitution of alanine for the asparagine at position 134 at the C-terminus of the intein or a substitution of alanine or serine for the cysteine at position 1 at the N-terminus of the intein.

## Patentansprüche

1. Verfahren zum Ligieren von Targetproteinen, **dadurch gekennzeichnet, dass** es umfasst:
(a) Exprimieren, von einem ersten Plasmid, eines ersten Fusionsproteins mit einem ersten Targetprotein mit einem C-Terminus, der an ein Intein fusioniert ist;
(b) Exprimieren eines zweiten Fusionsproteins mit einem zweiten Targetprotein mit einem N-terminalen Cystein, fusioniert an ein Intein, von einem zweiten Plasmid;
(c) Erhalten einer Zubereitung des ersten Fusionsproteins und einer Zubereitung des zweiten Fusionsproteins;
(d) Zugeben eines Thiol-Reagens zur Zubereitung des ersten Fusionsproteins, so dass das erste Intein geschnitten wird, um einen C-terminalen Thioester auf dem ersten Targetprotein auszubilden;
(e) Schneiden des zweiten Inteins aus dem zweiten Targetprotein in der Zubereitung des zweiten Fusionsproteins und Ausbilden eines N-terminalen Cysteins auf dem zweiten Targetprotein;
und
(f) Zulassen der Ligierung des ersten Targetproteins mit dem C-terminalen Thioester mit dem zweiten Targetprotein aus (e);
wobei die Inteine entweder N- und/oder C-terminale Schnitteigenschaften aufweisen.

2. Verfahren nach Anspruch 1, wobei das erste Intein das Methanobacterium thermoautotrophicum (Mth) RIR1-Intein, dargestellt in SEQ ID NO: 24, ist.

3. Verfahren nach Anspruch 1, wobei das zweite Intein das Mth RIR1-Intein, dargestellt in SEQ ID NO: 24, ist.

4. Verfahren nach Anspruch 2 oder Anspruch 3, wobei die weitere Modifikation des Mth RIR1-Inteins eine Substitution von Alanin für das Asparagin an der Position 134 am C-Terminus oder eine Substitution von Alanin oder Serin für das Cystein an der Postion 1 am N-Terminus umfasst.

5. Verfahren nach Anspruch 1, wobei das zweite Targetprotein aus (e) aus dem zweiten Intein in Gegenwart eines Thiol-Reagens oder durch Modulierung der Temperatur, des pH-Werts, des Salzes, chaotroper Mittel oder von Kombinationen hiervon erfolgt.

6. Verfahren nach Anspruch 1, wobei (c) weiterhin das Aufreinigen des ersten oder zweiten Fusionsproteins aus der Zubereitung umfasst.

7. Verfahren nach Anspruch 6, wobei die Aufreinigung des Fusionsproteins weiterhin die Bindung an eine Chitinharzsäule umfasst.

8. Verfahren nach Anspruch 1, wobei die ersten und zweiten Plasmide zur Expression in einer Wirtszelle befähigt sind, ausgewählt aus einer Bakterien-, einer Hufe-, einer Pflanzen-, einer Insekten- und einer Säugerwirtszelle.

9. Verfahren zur Zyklisierung eines Targetproteins mit einem N-terminalen Cystein, **dadurch gekennzeichnet, dass** es umfasst:
(a) Exprimieren aus einem Plasmid, in einer Wirtszelle von einem Fusionsprotein, umfassend ein Targetprotein mit einem Cystein am N-Terminus, und zwei Inteinen, wobei das erste Intein an den C-Terminus des Targetproteins fusioniert ist und das zweite Intein an das N-terminale Cystein des Targetproteins fusioniert ist;
(b) Erhalten einer Zubereitung des exprimierten Fusionsproteins;
(c) Induzieren des Schneidens des Fusionsproteins, umfassend die Zugabe eines Thiolreagens, um die ersten und zweiten Inteine aus dem Targetprotein zu entfernen, um hierdurch das Targetprotein mit einem C-terminalen Thioester und einem N-terminalen Cystein zu erhalten;
und
(d) Zulassen einer intramolekularen Ligierung des N-Terminus des Targetproteins an den C-Terminus des Targetprotein, um hierdurch ein zyklisiertes Protein auszubilden;
wobei die Inteine entweder N- und/oder C-terminale Schnitteigenschaften aufweisen.

10. Verfahren nach Anspruch 9, wobei das Intein das Mth RIR1-Intein, dargestellt in SEQ ID NO:24, ist, oder eine weitere modifizierte Form des Mth RIR1-Inteins, wobei die Modifikation eine Substitution von Alanin für das Asparagin an der Position 134 am C-Terminus oder einer Substitution von Alanin oder Serin für das Cystein an der Position 1 des N-Terminus umfasst.

11. Verfahren nach Anspruch 9, wobei (i) die Modulation eines von Temperatur, pH-Wert, Salz, der Konzentration chaotroper Mittel oder Kombinationen hiervon das Intein aus dem Targetprotein schneidet, um das N-terminale Cystein auszubilden, und (ii) die Zugabe des Thiolreagens den C-terminalen Thioester auf dem Targetprotein ausbildet.

12. Verfahren nach Anspruch 9, wobei (b) weiterhin umfasst: Aufreinigen des Fusionsproteins aus der Zubereitung.

13. Verfahren nach Anspruch 9, wobei die Aufreinigung des Fusionsproteins weiterhin das Binden an eine Chitinharzsäule umfasst.

14. Verfahren nach Anspruch 9, wobei das Plasmid zur Expression in einer Wirtszelle befähigt ist, ausgewählt aus einer Bakterien-, einer Hefe-, einer Pflanzen-, einer Insekten- und einer Säugerwirtszelle.

15. Verfahren zum Ausbilden eines Polymers durch intermolekulare Ligierung zwischen Targetproteinen in einer Zubereitung, **dadurch gekennzeichnet, dass** es umfasst:
(a) Ausbilden jeweils des Targetproteins mit einem C-terminalen Thioester und einem N-terminalen Cystein durch Schneiden eines ersten und zweiten Inteins aus einem Fusionsprotein, wobei das Fusionsprotein ein Targetproteins umfasst, welches an das erste Intein am C-terminalen Ende und an das zweite Intein am N-terminalen Ende fusioniert ist;
und
(b) Zulassen einer intermolekularen Ligierung zwischen den Targetproteinen durch Umsetzen des C-terminalen Thioesters des einen Targetproteins mit dem N-terminalen Cystein am N-Terminus des anderen Targetproteins, um ein Polymer auszubilden;
wobei die Inteine entweder N- und/oder C-terminale Schneideigenschaften besitzen.

16. Verfahren nach Anspruch 15, wobei das erste Intein das Mth RIR1-Intein, dargestellt in SEQ ID NO:24, ist.

17. Verfahren nach Anspruch 15, wobei das zweite Intein das Mth RIR1-Intein, dargestellt in SEO ID NO:24, ist.

18. Verfahren nach Anspruch 16 oder 17, wobei die weitere Modifizierung des Mth RIR1-Inteins eine Substitution von Alanin für das Asparagin an der Position 134 am C-Terminus oder eine Substitution von Alanin oder Serin für das Cystein an der Position 1 am N-Terminus umfasst.

19. Verfahren nach Anspruch 15, wobei das zweite Intein, aus dem Targetprotein durch Modulieren der Temperatur, des pH-Werts, des Salzes oder chaotropes Mittel order Kombinationen hiervon geschnitten wird.

20. Verfahren nach Anspruch 15, wobei das Fusionsprotein exprimiert wird durch ein Plasmid, welches zu einer Expression in einer Wirtszelle befähigt ist, ausgewählt aus einer Bakterien-, einer Hefe-, einer Pflanzen-, einer Insekten- und einer Säugerwirtszelle.

21. Verfahren zur Bindung eines Cysteins am N-Terminus eines Targetproteins, **dadurch gekennzeichnet, dass** es umfasst:
Exprimieren einer für ein Fusionsprotein codierenden Nucleinsäure in einer Wirtszelle, umfassend ein Intein und ein Targetprotein, wobei die Intein-kodierende Sequenz 5' zu einem Codon liegt, welches ein Cystein am Aminoterminus des Targetproteins spezifiziert;
und
Schneiden des Inteins aus dem Targetprotein, um das Cystein am N-Terminus des Targetproteins zu generieren;
wobei die Inteine entweder N- und/oder C-Terminale Schnitteigenschaften besitzen.

22. Verfahren zum Ligieren eines ersten und eines zweiten Targetproteins, **dadurch gekennzeichnet, dass** es umfasst:
(a) Induzieren des Schneidens eines ersten Inteins aus einem Fusionsprotein, umfassend das Intein und ein erstes Targetprotein, um auf dem Targetprotein ein Cystein am N-Terminus auszubilden;
(b) Kombinieren in einer Mischung des ersten Targetproteins aus (a) mit einem zweiten Targetprotein mit einem C-Terminus Thioester; und
(c) Ligieren der ersten und zweiten Targetproteine;
wobei die Inteine entweder N- und/oder C-terminale Schnitteigenschaften besitzen.

23. Verfahren zum Zyklisieren eines Targetproteins mit einem N-terminalen Cystein, **dadurch gekennzeichnet, dass** es umfasst:
Zugeben eines Thiolreagens zu einem Fusionsproteins umfassend ein Targetprotein mit einem N-terminalen Cystein und ein Intein, welches an den C-Terminus des Targetproteins fusioniert ist, um das Schneiden des Inteins aus dem Targetprotein und die Ausbildung eines C-terminalen Thioesters auf dem Targetprotein zu induzieren; und Zulassen einer intramolekularen Ligierung des N-Terminus des C-terminalen Thioesters des Targetproteins an das N-terminale Cystin des Targetproteins zur Zyklisierung des Targetproteins;
wobei die Inteine entweder N- und/oder C-terminale Schnitteigenschaften aufweisen.

24. Ein weiter modifiziertes Mth RIR1-Intein, **dadurch gekennzeichnet, dass** das Intein die in SEO ID NO:24 dargestellte Aminosäuresequenz aufweist, die eine Substitution von Alanin für das Asparagin an der Position 134 am C-Terminus des Inteins oder eine Substitution von Alanin oder Serin für das Cystein an der Position 1 am N-Terminus des Inteins aufweist.

## Revendications

1. Procédé pour ligaturer des protéines cibles, **caractérisé en ce qu'**il comprend :
(a) l'expression, à partir d'un premier plasmide, d'une première protéine de fusion comprenant une première protéine cible ayant une extrémité C fusionnée à une intéine ;
(b) l'expression, à partir d'un second plasmide, d'une seconde protéine de fusion comprenant une seconde protéine cible ayant une cystéine N-terminale fusionnée à une intéine ;
(c) l'obtention d'une préparation à partir de la première protéine de fusion et d'une préparation à partir de la seconde protéine de fusion ;
(d) l'addition d'un réactif thiol à la préparation de la première protéine de fusion de manière à ce que la première intéine soit clivée pour former un thioester C-terminal sur la première protéine cible ;
(e) le clivage de la seconde intéine à partir de la seconde protéine cible dans la préparation de la seconde protéine de fusion et la formation d'une cystéine N-terminale sur la seconde protéine cible ;
et
(f) le fait de permettre la ligature de la première protéine cible ayant le thioester C-terminal avec la seconde protéine cible du point (e) ;
les intéines présentant un clivage N-terminal et/ou C-terminal.

2. Procédé selon la revendication 1, dans lequel la première intéine est l'intéine RIR1 de *Methanobacterium thermoautotrophicum* (Mth) illustrée dans la SEQ ID NO: 24.

3. Procédé selon la revendication 1, dans lequel la seconde intéine est l'intéine RIR1 de Mth illustrée dans la SEQ ID NO: 24.

4. Procédé selon la revendication 2 ou la revendication 3, dans lequel l'autre modification de l'intéine RIR1 de Mth comprend une substitution de l'asparagine par l'alanine en position 134 à l'extrémité C ou une substitution de la cystéine par l'alanine ou la sérine en position 1 à l'extrémité N.

5. Procédé selon la revendication 1, dans lequel la seconde protéine cible du point (e) est clivée à partir de la seconde intéine en présence d'un réactif thiol ou en modulant n'importe quel paramètre parmi la température, le pH, le sel, les agents chaotropes ou une de leurs combinaisons.

6. Procédé selon la revendication 1, dans lequel le point (c) comprend en outre la purification de la première ou de la seconde protéine de fusion de la préparation.

7. Procédé selon la revendication 6, dans lequel la purification de la protéine de fusion comprend en outre la fixation à une colonne garnie de résine couplée à de la chitine.

8. Procédé selon la revendication 1, dans lequel les premier et second plasmides sont capables de s'exprimer dans une cellule hôte choisie parmi une cellule hôte de bactérie, de levure, de plante, d'insecte et de mammifère.

9. Procédé pour la cyclisation d'une protéine cible ayant une cystéine N-terminale, **caractérisé en ce qu'**il comprend :
(a) l'expression, à partir d'un plasmide dans une cellule hôte, d'une protéine de fusion comprenant une protéine cible ayant une cystéine à l'extrémité N et deux intéines, la première intéine ayant fusionné avec l'extrémité C de la protéine cible et la seconde intéine ayant fusionné avec la cystéine N-terminale de la protéine cible ;
(b) l'obtention d'une préparation de la protéine de fusion exprimée ;
(c) l'induction du clivage de la protéine de fusion comprenant l'addition d'un réactif thiol pour éliminer les première et seconde intéines de la protéine cible et ainsi obtenir la protéine cible ayant un thioester C-terminal et une cystéine N-terminale ;
et
(d) le fait de permettre la ligature intramoléculaire de l'extrémité N de la protéine cible avec l'extrémité C de la protéine cible et ainsi former une protéine cyclisée, les intéines présentant un clivage N-terminal et/ou C-terminal.

10. Procédé selon la revendication 9, dans lequel l'intéine est l'intéine RIR1 de Mth illustrée dans la SEQ ID NO: 24 ou une autre forme modifiée de l'intéine RIR1 de Mth dans laquelle la modification comprend une substitution de l'asparagine par l'alanine en position 134 à l'extrémité C ou une substitution de la cystéine par l'alanine ou la sérine en position 1 à l'extrémité N.

11. Procédé selon la revendication 9, dans lequel (i) la modulation d'un paramètre quelconque comme la température, le pH, le sel, la concentration des agents chaotropes ou une de leurs combinaisons clive l'intéine de la protéine cible pour former la cystéine N-terminale et (ii) l'addition du réactif thiol forme le thioester C-terminal sur la protéine cible.

12. Procédé selon la revendication 9, dans lequel le point (b) comprend en outre la purification de la protéine de fusion de la préparation.

13. Procédé selon la revendication 9, dans lequel la purification de la protéine de fusion comprend en outre la fixation à une colonne garnie de résine couplée à de la chitine.

14. Procédé selon la revendication 9, dans lequel le plasmide est capable de s'exprimer dans une cellule hôte choisie parmi une cellule hôte de bactérie, de levure, de plante, d'insecte et de mammifère.

15. Procédé pour former un polymère par ligature intramoléculaire entre les protéines cibles dans une préparation, **caractérisé en ce qu'**il comprend :
(a) la formation de chaque protéine cible ayant un thioester C-terminal et une cystéine N-terminale en clivant les première et seconde intéines à partir d'une protéine de fusion, la protéine de fusion comprenant une protéine cible ayant fusionné avec la première intéine à l'extrémité C et avec la seconde intéine à l'extrémité N ;
et
(b) le fait de permettre une ligature intermoléculaire entre les protéines cibles en faisant réagir le thioester C-terminal d'une première protéine cible avec la cystéine N-terminale à l'extrémité N d'une autre protéine cible pour former un polymère ;
les intéines présentant un clivage N-terminal et/ou C-terminal.

16. Procédé selon la revendication 15, dans lequel la première intéine est l'intéine RIR1 de Mth illustrée dans la SEQ ID NO: 24.

17. Procédé selon la revendication 15, dans lequel la seconde intéine est l'intéine RIR1 de Mth illustrée dans la SEQ ID NO: 24.

18. Procédé selon la revendication 16 ou la revendication 17, dans lequel l'autre modification de l'intéine RIR1 de Mth comprend une substitution de l'asparagine par l'alanine en position 134 à l'extrémité C ou une substitution de la cystéine par l'alanine ou la sérine en position 1 à l'extrémité N.

19. Procédé selon la revendication 15, dans lequel la seconde intéine est clivée à partir de la protéine cible en modulant la température, le pH, le sel ou les agents chaotropes ou une de leurs combinaisons.

20. Procédé selon la revendication 15, dans lequel la protéine de fusion est exprimée par un plasmide qui est capable de s'exprimer dans une cellule hôte choisie parmi une cellule hôte de bactérie, de levure, de plante, d'insecte et de mammifère.

21. Procédé pour générer une cystéine à l'extrémité N d'une protéine cible, **caractérisé en ce qu'**il comprend :
l'expression, dans une cellule hôte, d'un acide nucléique codant pour une protéine de fusion comprenant une intéine et une protéine cible, dans lequel la séquence codant pour l'intéine est 5' jusqu'à un codon spécifiant une cystéine à l'extrémité amino de la protéine cible ;
et
le clivage de l'intéine à partir de la protéine cible de manière à générer la cystéine à l'extrémité N de la protéine cible ;
les intéines présentant un clivage N-terminal et/ou C-terminal.

22. Procédé pour ligaturer une première et une seconde protéine cible, **caractérisé en ce qu'**il comprend :
(a) l'induction d'un clivage d'une première intéine à partir d'une protéine de fusion comprenant l'intéine et une première protéine cible pour former une cystéine N-terminale sur la protéine cible ;
(b) la combinaison dans un mélange de la première protéine cible du point (a) et d'une seconde protéine cible ayant un thioester C-terminal ;
et
(c) la ligature des première et seconde protéines cibles ;
les intéines présentant un clivage N-terminal et/ou C-terminal.

23. Procédé pour la cyclisation d'une protéine cible ayant une cystéine N-terminale, **caractérisé en ce qu'**il comprend :
l'addition d'un réactif thiol à une protéine de fusion comprenant une protéine cible ayant une cystéine N-terminale et une intéine fusionnée à l'extrémité C de la protéine cible afin d'induire le clivage de l'intéine à partir de la protéine cible et la formation d'un thioester C-terminal sur la protéine cible ;
et
le fait de permettre la ligature intramoléculaire de l'extrémité N du thioester C-terminal de la protéine cible avec la cystéine N-terminale de la protéine cible pour effectuer la cyclisation de la protéine cible ;
les intéines présentant un clivage N-terminal et/ou C-terminal.

24. Intéine RIR1 de Mth davantage modifiée, **caractérisée en ce que** l'intéine comprenant la séquence d'acides aminés illustrée dans la SEQ ID NO: 24 comprend une substitution de l'asparagine par l'alanine en position 134 à l'extrémité C de l'intéine ou une substitution de la cystéine par l'alanine ou la sérine en position 1 à l'extrémité N de l'intéine.


### SEQUENCE LISTING

<110> Evans, Thomas
   Xu, Ming-Qun
   NEW ENGLAND BIOLABS, INC.
<120> Intein-Mediated Protein Ligation Of Expressed Proteins
<130> NEB-154-PCT
<140>
<141>
<150> 09/249, 543
   <151> 1999-02-12
<160> 24
<170> PatentIn Ver. 2.0
<210> 1
   <211> 99
   <212> DNA
   <213> Artificial Sequence
<220>
   <223> Description of Artificial Sequence: Chemically Synthesized From Methanobacterium thermoautotrophicum.
<400> 1
   tcgaggcaac caacccctgc gtatccggtg acaccattgt aatgactagt ggcggtccgc 60
   gcactgtggc tgaactggag ggcaaaccgt tcaccgcac                        99
<210> 2
   <211> 93
   <212> DNA
   <213> Artificial Sequence
<220>
   <223> Description of Artificial Sequence: Chemically Synthesized From Methanobacterium thermoautotrophicum.
<400> 2
   ccggttggct gctcgccaca gttgtgtaca atgaagccat tagcagtgaa tgcgctagca 60
   ccgtaaacag tagcgtcata aacatcctgg cgg                              93
<210> 3
   <211> 100
   <212> DNA
   <213> Artificial Sequence
<220>
   <223> Description of Artificial Sequence: Chemically Synthesized From Methanobacterium thermoautotrophicum.
<400> 3
   tgattcgcgg ctctggctac ccatgcccct caggtttctt ccgcacctgt gaacgtgacg 60
   tatatgatct gcgtacacgt gagggtcatt gcttacgttt 100
<210> 4
   <211> 100
   <212> DNA
   <213> Artificial Sequence
<220>
   <223> Description of Artificial Sequence: Chemically Synthesized From Methanobacterium thermoautotrophicum.
<400> 4
   gacccatgat caccgtgttc tggtgatgga tggtggcctg gaatggcgtg ccgcgggtga 60
   actggaacgc ggcgaccgcc tggtgatgga tgatgcagct 100
<210> 5
   <211> 87
   <212> DNA
   <213> Artificial Sequence
<220>
   <223> Description of Artificial Sequence: Chemically Synthesized From Methanobacterium thermoautotrophicum.
<400> 5
   ggcgagtttc cggcactggc aaccttccgt ggcctgcgtg gcgctggccg ccaggatgtt 60
   tatgacgcta ctgtttacgg tgctagc 87
<210> 6
   <211> 49
   <212> DNA
   <213> Artificial Sequence
<220>
   <223> Description of Artificial Sequence: Chemically Synthesized From Methanobacterium thermoautotrophicum.
<400> 6
   gcattcactg ctaatggctt cattgtacac aactgtggcg agcagccaa 49
<210> 7
   <211> 100
   <212> DNA
   <213> Artificial Sequence
<220>
   <223> Description of Artificial Sequence: Chemically Synthesized From Methanobacterium thermoautotrophicum.
<400> 7
   ccagcgccac gcaggccacg gaaggttgcc agtgccggaa actcgccagc tgcatcatcc 60
   atcaccaggc ggtcgccgcg ttccagttca cccgcggcac 100
<210> 8
   <211> 90
   <212> DNA
   <213> Artificial Sequence
<220>

   <223> Description of Artificial Sequence: Chemically Synthesized From Methanobacterium thermoautotrophicum.
<400> 8
   gccattccag gccaccatcc atcaccagaa cacggtgatc atgggtcaaa cgtaagcaat 60
   gaccctcacg tgtacgcaga tcatatacgt 90
<210> 9
   <211> 97
   <212> DNA
   <213> Artificial Sequence
<220>
   <223> Description of Artificial Sequence: Chemically Synthesized From Methanobacterium thermoautotrophicum.
<400> 9
   cacgttcaca ggtgcggaag aaacctgagg ggcatgggta gccagagccg cgaatcagtg 60
   cggtgaacgg tttgccctcc agttcagcca cagtgcg 97
<210> 10
   <211> 55
   <212> DNA
   <213> Artificial Sequence
<220>
   <223> Description of Artificial Sequence: Chemically Synthesized From Methanobacterium thermoautotrophicum.
<400> 10
   cggaccgcca ctagtcatta caatggtgtc accggatacg caggggttgg ttgcc 55
<210> 11
   <211> 45
   <212> DNA
   <213> Artificial Sequence
<220>
   <223> Description of Artificial Sequence: Chemically Synthesized From Methanobacterium thermoautotrophicum.
<400> 11
   tcgaggcaac caacgcatgc gtatccggtg acaccattgt aatga 45
<210> 12
   <211> 45
   <212> DNA
   <213> Artificial Sequence
<220>
   <223> Description of Artificial Sequence: Chemically Synthesized From Methanobacterium thermoautotrophicum.
<400> 12
   ctagtcatta caatggtgtc accggatacg catgcgttgg ttgcc 45
<210> 13
   <211> 36
   <212> DNA
   <213> Artificial Sequence
<220>
   <223> Description of Artificial Sequence: Chemically Synthesized From Methanobacterium thermoautotrophicum.
<400> 13
   tcgagggctg cgtatccggt gacaccattg taatga 36
<210> 14
   <211> 36
   <212> DNA
   <213> Artificial Sequence
<220>
   <223> Description of Artificial Sequence: Chemically Synthesized From Methanobacterium thermoautotrophicum.
<400> 14
   ctagtcatta caatggtgtc accggatacg cagccc 36
<210> 15
   <211> 54
   <212> DNA
   <213> Artificial Sequence
<220>
   <223> Description of Artificial Sequence: Chemically Synthesized From Methanobacterium thermoautotrophicum.
<400> 15
   tcgagggcat cgaggcaacc aacggatccg tatccggtga caccattgta atga 54
<210> 16
   <211> 54
   <212> DNA
   <213> Artificial Sequence
<220>
   <223> Description of Artificial Sequence: Chemically Synthesized From Methanobacterium thermoautotrophicum.
<400> 16
   ctagtcatta caatggtgtc accggatacg gatccgttgg ttgcctcgat gccc 54
<210> 17
   <211> 54
   <212> DNA
   <213> Artificial Sequence
<220>
   <223> Description of Artificial Sequence: Chemically Synthesized From Methanobacterium thermoautotrophicum.
<400> 17
   tcgagggcat cgaggcaacc aacggcgccg tatccggtga caccattgta atga 54
<210> 18
   <211> 54
   <212> DNA
   <213> Artificial Sequence
<220>
   <223> Description of Artificial Sequence: Chemically Synthesized From Methanobacterium thermoautotrophicum.
<400> 18
   ctagtcatta caatggtgtc accggatacg gcgccgttgg ttgcctcgat gccc 54
<210> 19
   <211> 28
   <212> DNA
   <213> Artificial Sequence
<220>
   <223> Description of Artificial Sequence: Chemically Synthesized From Methanobacterium thermoautotrophicum.
<400> 19
   gtacacgcat gcggcgagca gcccggga 28
<210> 20
   <211> 28
   <212> DNA
   <213> Artificial Sequence
<220>
   <223> Description of Artificial Sequence: Chemically Synthesized From Methanobacterium thermoautotrophicum.
<400> 20
   ccggtcccgg gctgctcgcc gcatgcgt 28
<210> 21
   <211> 14
   <212> PRT
   <213> Artificial Sequence
<220>
   <223> Description of Artificial Sequence: Chemically Synthesized From Methanobacterium thermoautotrophicum.
<220>
   <223> At position 12, "Xaa" = any amino acid
<400> 21
   Thr Leu Glu Gly Cys Gly Glu Gln Pro Thr Gly Xaa Leu Lys
    1               5                   10
<210> 22
   <211> 7
   <212> PRT
   <213> Artificial Sequence
<220>
   <223> Description of Artificial Sequence: Chemically Synthesized From Methanobacterium thermoautotrophicum.
<400> 22
   Cys Gly Glu Gln Pro Thr Gly
    1               5
<210> 23
   <211> 462
   <212> DNA
   <213> Artificial Sequence
<220>
   <223> Description of Artificial Sequence: Chemically Synthesized From Methanobacterium thermoautotrophicum.
<400> 23
   caactcggga ggatagaggc aaccaacccc tgtgtatccg gtgacaccat tgtaatgaca 60
   tccgggggtc cgcggacagt ggctgaactg gagggcaagc ccttcaccgc acttatcagg 120
   ggctcagggt acccctgccc ctcaggtttc ttcaggacct gtgaacggga cgtatatgat 180
   cttagaacca gggagggtca ttgcttaagg ttgacccatg atcacagggt ccttgtaatg 240
   gatggtggtc tggaatggcg tgccgccggt gaacttgaaa ggggagaccg ccttgtgatg 300
   gatgatgctg caggggagtt tccggcactt gcaaccttca gaggcctcag gggcgccggc 360
   cgccaggatg tctatgacgc cactgtctac ggtgccagtg cattcacagc caatggattc 420
   atagtccaca actgtgggga gcagccactc ctcacccatg aa                    462
<210> 24
   <211> 447
   <212> DNA
   <213> Artificial Sequence
<220>
   <223> Description of Artificial Sequence: Chemically Synthesized From Methanobacterium thermoautotrophicum.
<400> 24
   ctcgaggcaa ccaacccctg cgtatccggt gacaccattg taatgactag tggcggtccg 60
   cgcactgtgg ctgaactgga gggcaaaccg ttcaccgcac tgattcgcgg ctctggctac 120
   ccatgcccct caggtttctt ccgcacctgt gaacgtgacg tatatgatct gcgtacacgt 180
   gagggtcatt gcttacgttt gacccatgat caccgtgttc tggtgatgga tggtggcctg 240
   gaatggcgtg ccgcgggtga actggaacgc ggcgaccgcc tggtgatgga tgatgcagct 300
   ggcgagtttc cggcactggc aaccttccgt ggcctgcgtg gcgctggccg ccaggatgtt 360
   tatgacgcta ctgtttacgg tgctagcgca ttcactgcta atggcttcat tgtacacaac 420
   tgtggcgagc agccaaccgg tgaattc                                     447